# EUROPEAN PATENT APPLICATION

(11) **EP 2 821 079 A1**
(43) Date of publication of application: **07.01.2015**
(21) Application number: 13754232.0
(22) Date of filing: 28.02.2013
(51) Int. Cl.: A61K 38/21, A61P 1/16, A61P 13/12, A61P 35/00, A61P 43/00

(54) **BODY CAVITY EFFUSION SUPPRESSANT**

(30) Priority: 29.02.2012 JP 2012043702
(71) Applicant: Toray Industries, Inc., Tokyo 103-8666 (JP)
(72) Inventor: NARUMI, Hideki, Kamakura-shi Kanagawa 248-8555 (JP); IKEHARA, Sanae, Tsukuba-shi Ibaraki 305-0035 (JP)
(74) Representative: Kador & Partner
(86) International application number: PCT/JP2013/055331
(87) International publication number: WO 2013/129549

(57) **Abstract**

The object of the present invention is to provide an inhibitory agent for body cavity fluid accumulation, which exerts a drug efficacy on body cavity fluid accumulation that is resistant to administration of diuretics, and is capable of exerting therapeutic effects even by systemic administration. The present invention provides an inhibitory agent for body cavity fluid accumulation comprising, as an active ingredient, a covalent conjugate of interferon with polyalkylene glycol.

## Description

### Technical Field

The present invention relates to an inhibitory agent for body cavity fluid accu mulation.

### Background Art

A large quantity of water exists in an animal body, and extracellular fluid that mainly fills spaces between tissues or in the body cavity, ducts, or circulatory systems (e.g., tissue fluid, body cavity fluid, blood, or lymph) is referred to as "body fluid." In particular, fluid existing in the body cavity is referred to as a "body cavity fluid" (e.g., a pleural effusion, ascites, or pericardial effusion). While small amounts of body cavity fluids exist in a healthy person, it is known that large amounts of body cavity fluids are retained in the body of a patient with, for example, a hepatic disease, a renal disease, a cardiac disease, cancer, or an inflammatory disease. As body cavity fluid accumulation progresses, symptoms such as chest pain, abdominal pain, feeling of fullness, and respiratory difficulty develop. Since such symptoms remarkably decrease the quality of life (QOL) of a patient, a treatment for removing body cavity fluid at an early stage is necessary.

While the mainstream treatment for body cavity fluid accumulation is drug therapy with administration of diuretics, a tube is inserted in the body cavity to directly remove a body cavity fluid via suction in cases with severe symptoms. In clinical studies, a high-dose interferon-α or interferon-β is topically administered to the body cavity of a patient with, for example, digestive system cancer or ovarian cancer, in an attempt to inhibit accumulation of a cancerous (or malignant) pleural effusion or ascites (Non-Patent Documents 1 to 3). However, effects of inhibiting body cavity fluid accumulation that can be useful in clinical application have not been achieved.

Concerning interferon that is used as a therapeutic agent for a virus-mediated hepatic disease, multiple sclerosis, or cancer, interferon preparations with improved *in-vivo* sustainability achieved via covalent binding of interferon to polyethylene glycol have been developed (Patent Documents 1 to 6 and Non-Patent Documents 4 and 5).

### Citation List

### Patent Documents:

Patent Document 1: US Patent No. 4,917,888
Patent Document 2: International Publication WO 1987/00056
Patent Document 3: International Publication WO 1999/55377
Patent Document 4: International Publication WO 2000/23114
Patent Document 5: JP H9-25298 A (1997)
Patent Document 6: JP Patent No. 4,850,514

### Non-Patent Documents:

Non-Patent Document 1: Gavin et al., Cancer, (1993) Vol. 71, pp. 2027-2030
Non-Patent Document 2: Wakui et al., Biotherapy, (1988) Vol. 2, pp. 339-347
Non-Patent Document 3: Gebbia et al., In Vivo, (1991) Vol. 5, pp. 579-582
Non-Patent Document 4: Pepinsky et al., The Journal of Pharmacology and Experimental Therapeutics, (2001) Vol. 297, pp. 1059-1066
Non-Patent Document 5: Bailon et al., Bioconjugate Chemistry, (2001) Vol. 12, pp. 195-202

### Summary of the Invention

### Problem to Be Solved by the Invention

However, the use of diuretics for the purpose of treatment of body cavity fluid accumulation has a risk of causing an electrolyte imbalance in a patient to which such diuretics have been administered, and thereby causing a feeling of unwellness. In that case, there is a problem that, if the excretion of urine becomes difficult, body cavity fluid accumulation is aggravated. In addition, the effects of diuretics for body cavity fluid removal are insufficient in cases of peritoneal dissemination or ascites, and therapeutic effects thereof are limited. Despite the large burden imposed on a patient because of puncture, the removal of body cavity fluid via direct suction merely has a transient effect and further has a problem that it makes nutritional conditions or immune conditions become deteriorated due to a loss of albumin and globulin.

In cases of malignant pleural effusion and ascites, also, neither single administration of interferon-β (Non-Patent Document 3), intermittent administration of interferon-α (Non-Patent Document 1), or intraperitoneal (i.e., topical) administration of interferon-α has resulted in inhibition of body cavity fluid amount at clinically remarkable levels. In cases of malignant ascites, further, topical administration of 6,000,000 units of interferon-β at three times a week, which is also employed for other indications, has not yielded any substantial therapeutic effects. Further, daily and topical administration at a dose of 18,000,000 units resulted in only response rate as low as less than 50% (Non-Patent Document 2). Even when interferon was topically administered into the body cavity continuously, accordingly, it was impossible to attain effects of inhibiting body cavity fluid accumulation that could be useful in clinical application.

Accordingly, it is an object of the present invention to provide an inhibitory agent for body cavity fluid accumulation, which exerts drug efficacy on body cavity fluid accumulation that is resistant to administration of diuretics, and is capable of exerting therapeutic effects even via systemic administration.

### Means for Solving the Problem

The present inventors have conducted concentrated studies in order to solve the problem. As a result, they found that a covalent conjugate of interferon with polyalkylene glycol had an excellent inhibitory effect on body cavity fluid accumulation, and completed the present invention.

Specifically, the present invention provides an inhibitory agent for body cavity fluid accumulation comprising, as an active ingredient, a covalent conjugate of interferon with polyalkylene glycol.

The polyalkylene glycol is preferably polyethylene glycol.

The interferon is preferably type I interferon or interferon-λ, and interferon-α or interferon-β is particularly preferred among various types of type I interferon.

The inhibitory agent for body cavity fluid accumulation preferably inhibits accumulation of a pleural effusion or ascites, and the pleural effusion or ascites is preferably a refractory pleural effusion or ascites accumulated due to cancer, cirrhosis, or renal failure.

In particular, the effects of diuretic administration cannot be expected in most cases of refractory pleural effusion or ascites, and therefore the clinical significance of the treatment using the inhibitory agent for body cavity fluid accumulation is considered to be remarkable.

The inhibitory agent for body cavity fluid accumulation can be topically administered into a body cavity, but is preferably used for systemic administration. In that case, the systemic administration is more preferably one selected from the group consisting of subcutaneous administration, intracutaneous administration, intramuscular administration, intravenous administration, and intraarterial administration.

By systemic administration, risks of incorrect puncture to visceral organs during topical administration into the body cavity, intracavital infection, and acceleration of metastasis in the presence of cancerous cells in the body cavity can be reduced. In comparison with intracavital administration, the range of medical facilities and health-care professionals that can perform the systemic administration is widened. In addition, intramuscular administration or subcutaneous administration can be carried out via self-injection.

While the inhibitory agent for body cavity fluid accumulation can be administered daily at an interval of once or twice a day, intermittent administration at an interval of once every two or more days is preferred, intermittent administration at an interval of once every two days to one month is more preferred, intermittent administration at an interval of once or twice a week to once a month is further preferred, and intermittent administration at an interval of once or twice a week is particularly preferred.

Further, the use of the inhibitory agent for body cavity fluid accumulation in combination with an antitumor agent is effective. In that case, the antitumor agent is preferably at least one antitumor agent selected from the group consisting of 5-FU, tegafur-uracil, tegafur-gimeracil-oteracil, and capecitabine.

### Effects of the Invention

The inhibitory agent for body cavity fluid accumulation according to the present invention is capable of inhibiting body cavity fluid accumulation and removing body cavity fluid via systemic administration, as well as via topical administration. Thus, the inhibitory agent for body cavity fluid accumulation according to the present invention can exert therapeutic effects on body cavity fluid accumulation and alleviate chest pain, abdominal pain, strong feeling of fullness, and respiratory difficulty resulting from body cavity fluid accumulation.

### Brief Description of the Drawings

Fig. 1 shows the effects of intraperitoneal administration of a covalent conjugate of interferon-β with polyethylene glycol for inhibiting accumulation of an ascites observed in gastric cancer peritoneal metastasis mouse models.
Fig. 2 shows the effects of subcutaneous administration of a covalent conjugate of interferon-β with polyethylene glycol for inhibiting accumulation of an ascites observed in gastric cancer peritoneal metastasis mouse models.
Fig. 3 shows the effects of subcutaneous administration of a covalent conjugate of interferon-α with polyethylene glycol for inhibiting accumulation of an ascites observed in gastric cancer peritoneal metastasis mouse models.
Fig. 4 shows the effects of intraperitoneal administration of a covalent conjugate of interferon-β with polyethylene glycol for inhibiting recurrence of accumulation of an ascites (i.e., reaccumulation after ascitic paracentesis) observed in gastric cancer peritoneal metastasis mouse models.
Fig. 5 shows the effects of subcutaneous administration of a covalent conjugate of interferon-β with polyethylene glycol for inhibiting recurrence of accumulation of an ascites (i.e., reaccumulation after ascitic paracentesis) observed in ovarian cancer peritoneal metastasis mouse models.
Fig. 6 shows the effects of subcutaneous administration of a covalent conjugate of interferon-β with polyethylene glycol for improving survival rate observed in ovarian cancer peritoneal metastasis mouse models.

### Embodiments for Carrying out the Invention

The inhibitory agent for body cavity fluid accumulation according to the present invention is characterized in that it comprises, as an active ingredient, a covalent conjugate of interferon with polyalkylene glycol.

One, or two or more molecules of polyalkylene glycol can bind to one molecule of interferon. The total molecular weight of polyalkylene glycol molecules to be bound to one molecule of interferon is preferably 5,000 to 240,000, more preferably 10,000 to 80,000, and further preferably 39,000 to 45,000. Herein, when two molecules of polyalkylene glycol each having a molecular weight of 20,000 are covalently bound to one molecule of interferon, this means that polyalkylene glycol with a molecular weight of 40,000 covalently binds to one molecule of interferon.

One, or two or more molecules of interferon can bind to one molecule of polyalkylene glycol. Even in this case, the molecular weight of one polyalkylene glycol molecule is preferably 5,000 to 520,000, more preferably 10,000 to 200,000, and further preferably 39,000 to 45,000.

One polyalkylene glycol molecule is composed of many repeat units, and molecular weight of polyalkylene glycol generally varies from individual molecule to individual molecule. Thus, a molecular weight of polyalkylene glycol is expressed as an average molecular weight. In this description, accordingly, the molecular weight of polyalkylene glycol means an average molecular weight.

As the polyalkylene glycol, a polymeric compound that is acceptable as a drug carrier, inactive or has extremely low activity on a living body and is non-toxic or has extremely low toxicity, can be preferably used.

Examples of the interferon include a protein having the same amino acid sequence as that of a naturally occurring interferon (hereafter, referred to as a "naturally occurring interferon"), an amino acid-mutant interferon having an amino acid sequence derived from the amino acid sequence of the naturally occurring interferon by deletion, substitution, or addition of one or several amino acids and having a biological activity of interferon (hereafter, referred to as "interferon activity"), an interferon with a sugar chain moiety modified from that of the naturally occurring interferon, and an interferon without a sugar chain moiety.

Examples of the interferon also include a recombinant interferon prepared via genetic engineering on the basis of the amino acid sequence or nucleotide sequence of a naturally occurring interferon.

The interferon can be obtained by conventional techniques such as extraction from tissue, protein synthesis via genetic engineering, or biological production using a natural or recombinant cell that expresses the interferon. A commercially available interferon can also be used as the interferon.

The interferon is preferably type I interferon or interferon-λ. Examples of type I interferon include interferon-α, interferon-β, interferon-ω, interferon-ε, and interferon-κ, and interferon-α or interferon-β is more preferred.

The term "covalent conjugate of interferon with polyalkylene glycol" refers to a conjugate of one, or two or more molecules of polyalkylene glycol covalently bound to one molecule of interferon and having interferon activity, or a conjugate of one, two or more molecules of interferon covalently bound to one molecule of polyalkylene glycol and having interferon activity. A conjugate of one, or two or more molecules of polyalkylene glycol covalently bound to one molecule of interferon and having interferon activity is preferred. The covalent conjugate of interferon with polyalkylene glycol may be a conjugate of interferon directly bound to polyalkylene glycol or a conjugate of interferon bound to polyalkylene glycol via a linker or the like.

The dose of the covalent conjugate of interferon with polyalkylene glycol can be expressed in interferon titer (Units; hereafter shown as "U") or a mass (g). A covalent conjugate of interferon with polyalkylene glycol preferably retains at least 10% of the specific activity of interferon (i.e., interferon activity per weight) compared with the interferon before it is covalently bound to the polyalkylene glycol.

In this description, the binding of a polymeric compound to a protein is also referred to as chemical modification or modification of a protein with a polymeric compound. The covalent conjugate of interferon with polyalkylene glycol is also referred to as an interferon chemically modified with polyalkylene glycol or simply as modified interferon. In particular, the covalent conjugate of interferon with polyethylene glycol (hereafter, abbreviated as "PEG") is also referred to as PEG-modified interferon, pegylated interferon, or PEG-bound interferon.

The interferon is covalently bound to polyalkylene glycol at, for example, an amino group, a thiol group, the N-terminus, the C-terminus, or a sugar chain of the interferon. Alternatively, a non-naturally-occurring amino acid that had been introduced into the interferon via a known genetic engineering technique can be used as a site of covalent binding.

Interferon activity of the covalent conjugate of interferon with polyalkylene glycol can be assayed via a known biological assay technique. Specifically, for determining interferon activity, cells that are highly sensitive to the interferon (e.g., the FL cell) can be treated with an analyte comprising an interferon, a given amount of infectious *Sindbis* virus, *Vesicular stomatitis* virus (abbreviated as "VSV") or the like can be added to the treated cells to infect the cells, and the degree of resistance against viruses induced by the interferon (hereafter, referred to as "anti-virus activity") can be assayed. Anti-virus activity is assayed using the inhibition of virus growth as an indicator, and it can be expressed as an interferon titer (U) based on the dilution factor of the analyte that inhibits 50% of the cytopathic effects (abbreviated as "CPE) to destroy the cells as a result of virus growth (Shigeyasu Kobayashi et al., "Menneki Seikagaku Jikkenhou in Zoku Seikagaku Jikken Koza 5 (Successive Course on Biochemical Experiment 5: Experimental Approaches in Immunobiochemistry))," Japanese Biochemical Soc. ed., Tokyo Kagaku Dojin, (1986) p. 245).

The covalent conjugate of interferon with polyalkylene glycol can be produced by a known technique. For example, a method for covalently binding polyalkylene glycol to an amino group of an interferon is described in US Patent No. 4,917,888 and International Publication WO 1987/00056. A method for covalently binding polyalkylene glycol to a thiol group of an interferon is described in International Publication WO 1999/55377. A method for covalently binding a polymer to the N terminus of an interferon is described in International Publication WO 2000/23114 and JP H09-25298 A (1997). A method for covalently binding polyalkylene glycol to a sugar chain of an interferon is described in Macromolecular Chemistry (1978) Vol. 179, p. 301, and US Patents No. 4,101,380 and 4,179,337. A method for covalently binding polyalkylene glycol to a non-naturally-occurring amino acid introduced into an interferon is described in Bioorganic & Medicinal Chemistry Letters (2004) Vol. 14, pp. 5743-5745. Covalent binding of Polyalkylene glycol to the C terminus of an interferon can be made by introducing cysteine or a non-naturally-occurring amino acid into the C terminus or in the vicinity thereof.

It is necessary to activate a terminus of polyalkylene glycol to be subjected to a covalent binding reaction depending on a method of forming a bond between an interferon and polyalkylene glycol. A polyalkylene glycol derivative comprising a terminus activated with a hydroxysuccinimide ester, nitrobenzenesulfonate ester, maleimide, ortho-pyridyl disulfide, vinyl sulfone, maleimide, iodoacetamide, carboxylic acid, azide, phosphine, or amine structure can be used for covalent binding of the interferon and polyalkylene glycol, and such polyalkylene glycol derivative can be synthesized with known techniques.

The covalent conjugate of interferon with polyalkylene glycol is preferably a covalent conjugate of interferon with PEG. In a preferred covalent conjugate of interferon with PEG, one PEG molecule with a molecular weight of 5,000 to 240,000, preferably 10,000 to 80,000, and more preferably 39,000 to 45,000 is covalently bound to one interferon molecule.

A preferred example of the covalent conjugate of interferon with PEG is a covalent conjugate of human interferon-β with PEG, which can be prepared by the method described in, for example, JP Patent No. 4,850,514. PEG is preferably bound to human interferon-β at the amino group of lysine at position 19 or 134 of the amino acid sequence of human interferon-β, and a covalent conjugate of one PEG molecule covalently bound to human interferon-βat that site is more preferred. A specific example is a covalent conjugate of one PEG molecule with a molecular weight of 40,000 or more (preferably 42,000) covalently bound to human interferon-β at the amino group of lysine at position 134 of its amino acid sequence.

The term "lysine at position 134 of the amino acid sequence of human interferon-β" used herein refers to an amino acid lysine which is located at position 134 from the N-terminal amino acid (methionine; designated as position 1) of the amino acid sequence of human interferon-β consisting of 166 amino acids (SEQ ID NO: 1). Herein, the position of an amino acid residue within an interferon is indicated as a position number relative to the N-terminal amino acid of interferon designated as position 1.

Another preferred example of the covalent conjugate of interferon with PEG is a covalent conjugate of human interferon-α with PEG. Specific examples include a covalent conjugate of interferon-α with PEG in which one branched PEG molecule with a molecular weight of about 40,000 is covalently bound to human interferon-α-2a at one of lysine residues (main sites: positions 31, 121, 131, and 134) via an amide bond (general name: Peginterferon alfa-2a); and a covalent conjugate of interferon-α with PEG in which one methoxy-PEG molecule with a molecular weight of about 12,000 is covalently bound to human interferon-α-2b at one of the amino acid residues (cysteine at position 1, histidine at position 7, lysine at position 31, histidine at position 34, lysine at position 49, lysine at position 83, lysine at position 112, lysine at position 121, tyrosine at position 129, lysine at position 131, lysine at position 133, lysine at position 134, serine at position 163, or lysine at position 164) via a carbonyl group (general name: Peginterferon-α-2b).

The term "body cavity" refers to a space in an animal body that has been formed in an isolated state from the exterior thereof, which includes thoracic cavity, abdominal cavity, and pericardial cavity. The term "body cavity fluid" refers to liquid that is present in a body cavity, and liquid in the thoracic cavity, the abdominal cavity, or the pericardial cavity is referred to as a pleural effusion, ascites, or pericardial effusion, respectively.

The term "body cavity fluid accumulation" refers to that there is more than a normal amount of a body cavity fluid in the body cavity. The term "inhibitory agent for body cavity fluid accumulation" refers to a drug that has an effect to inhibit accumulation of a body cavity fluid and remove a body cavity fluid.

The body cavity fluid accumulation-inhibiting effect of the inhibitory agent for body cavity fluid accumulation can be assayed using disease models of underlying diseases causing body cavity fluid accumulation (e.g., cancer). Examples of such disease models include gastric cancer peritoneal metastasis mouse models (Cancer Science, (2003) Vol. 94, pp. 112-118) and ovarian cancer peritoneal metastasis mouse models (Molecular cancer therapeutics, (2007) Vol. 6, pp. 2959-2966).

The inhibitory agent for body cavity fluid accumulation can be used for prevention or treatment of accumulation of a body cavity fluid, and it can also be used for inhibiting its recurrence after physical removal of the body cavity fluid. The inhibitory agent for body cavity fluid accumulation can be preferably used against accumulation of a pleural effusion or ascites.

Examples of underlying diseases that cause body cavity fluid accumulation include cancer, infectious disease (e.g., bacterial, tuberculous, fungal, atypical pneumonia, virus, or parasite infection), inflammatory disease (e.g., pancreatitis, sarcoidosis, or asbestos lung), connective tissue disease (e.g., rheumatism, systemic lupus erythematodes, Sjogren's syndrome, Wegener's granulomatosis, or Churg-Strauss syndrome), endocrine disease (e.g., myxedema), hepatic disease (e.g., cirrhosis), renal disease (e.g., nephrotic syndrome), and cardiac disease (e.g., congestive heart failure). The inhibitory agent for body cavity fluid accumulation can exert therapeutic effects on body cavity fluid accumulation caused by such diseases.

In addition, the inhibitory agent for body cavity fluid accumulation can also exert therapeutic effects on a refractory body cavity fluid accumulation, and it is particularly preferably used against a refractory pleural effusion or ascites accumulation. The term "refractory body cavity fluid accumulation" refers to a body cavity fluid accumulation that is difficult to control via medical treatment with e.g., diuretics (i.e., a diuretic-resistant or diuretic-intolerant), and includes, for example, a refractory pleural effusion or ascites accumulated due to cancer, cirrhosis, or renal failure.

Among refractory pleural effusions or refractory ascites, in particular, a pleural effusion and an ascites accumulated due to cancer are referred to as a cancerous (or malignant) pleural effusion and cancerous (or malignant) ascites, respectively, and their symptoms are known to be serious. However, the inhibitory agent for body cavity fluid accumulation can also exert therapeutic effects on accumulation of the malignant pleural effusion or malignant asicites. The malignant pleural effusion or or malignant asicites is generated due to intrathoracic or peritoneal metastasis of cancer or pleurisy or peritonitis caused by cancer.

The inhibitory agent for body cavity fluid accumulation is particularly preferably used for treatment or prevention of a malignant pleural effusion or malignant ascites among refractory pleural effusions or refractory ascites.

The inhibitory agent for body cavity fluid accumulation may be used in a mixture with other agents in an adequate amount or used in combination with other agents for the purpose of complementing or enhancing the effects of inhibiting body cavity fluid accumulation or reducing a dose.

Examples of drugs that can be used in combination with the inhibitory agent for body cavity fluid accumulation include those that are generally used for treatment of underlying diseases causing accumulation of a body cavity fluid. When a cancerous body cavity fluid accumulation is to be inhibited, for example, the inhibitory agent can be used in combination with an antitumor agent. Examples of preferred antitumor agents to be used in the combination include a molecular-targeting agent, an alkylating agent, an antimetabolite, a microtubule-inhibiting agent, a topoisomerase inhibitor, a platinum drug, and a carcinostatic antibiotic agent. More preferred examples of the antitumor agents are particularly cisplatin, carboplatin, irinotecan hydrochloride, 5-FU, tegafur-uracil, tegafur-gimeracil-oteracil, capecitabine, taxol, taxotere, imatinib, and sunitinib. Tegafur-gimeracil-oteracil potassium (tradename: TS-1^{®}) is a particularly preferable form of tegafur-gimeracil-oteracil. The inhibitory agent for body cavity fluid accumulation to be used in combination with the antitumor agent may be administered simultaneously with an antitumor agent. In order to enhance the effects of inhibiting ascites, alternatively, the inhibitory agent may be administered separately from an antitumor agent before or after treatment with the antitumor agent.

The inhibitory agent for body cavity fluid accumulation according to the present invention may be administered as a mixed preparation with the antitumor agent to a single site. Alternatively, these agents may be separately administered to the same site or different sites.

The dose (titer) and the frequency of administration of the inhibitory agent for body cavity fluid accumulation are adequately determined depending on age, body weight, and symptoms of the subject, dosage form, administration route, and the like. The agent can be daily administered to a human subject at a dose of 10,000 U to 18,000,000 U/dose at an interval of once or twice a day. Intermittent administration is preferably carried out at an interval of once every two or more days, more preferably at an interval of once every two days to one month, further preferably at an interval of once or twice a week to once a month, and particularly preferably at an interval of once or twice a week.

The inhibitory agent for body cavity fluid accumulation can be used as a medicine that is useful for treatment and prevention of body cavity fluid accumulation against a mammal (e.g., a mouse, rat, hamster, rabbit, dog, monkey, cow, sheep, or human).

In a clinical setting, the covalent conjugate of interferon with polyalkylene glycol may be used directly, or it may be used in the form of a pharmaceutical composition in mixture with a known pharmacologically acceptable carrier, excipient, or the like.

The inhibitory agent for body cavity fluid accumulation can be systemically or topically administered. Herein, the topical administration refers to administration at a site at which the direct effects are desired; that is, a site at which a body cavity fluid would accumulate or has accumulated. For example, intraperitoneal administration of the inhibitory agent for body cavity fluid accumulation against a ascites accumulation falls into the category of topical accumulation. The systemic administration refers to a mode of administration that allows a drug to be absorbed through the digestive system or a pathway other than the digestive system and provides the effects throughout the body (non-topically), at a site other than a site at which the direct effects are desired; that is, at a site other than a site at which a body cavity fluid would accumulate or has accumulated. Examples of systemic administration include oral administration, intraperitoneal administration (excluding in cases of ascites accumulation), subcutaneous administration, intracutaneous administration, intramuscular administration, intravenous administration, and intraarterial administration. It should be noted that intraperitoneal administration of the inhibitory agent for body cavity fluid accumulation against an ascites accumulation corresponds to topical administration. It is preferred that the inhibitory agent for body cavity fluid accumulation be administered systemically, and it is more preferred that the inhibitory agent be administered subcutaneously, intracutaneously, intramuscularly, intravenously, or intraarterially.

Preferred examples of the inhibitory agent for body cavity fluid accumulation include an inhibitory agent for body cavity fluid accumulation that comprises, as an active ingredient, a covalent conjugate of interferon with PEG, and is for use in systemic administration and intermittent administration at an interval of two or more days. In a more preferred example, the inhibitory agent for body cavity fluid accumulation comprises, as an active ingredient, a covalent conjugate of interferon-α or interferon-β with PEG, and is for use in systemic administration and intermittent administration at an interval of two or more days. In a further preferred example, the inhibitory agent for body cavity fluid accumulation comprises, as an active ingredient, a covalent conjugate of interferon-α or interferon-β with PEG, and is for use in subcutaneous administration and intermittent administration at an interval of two or more days. In a particularly preferred example, the inhibitory agent for body cavity fluid accumulation comprises, as an active ingredient, a covalent conjugate of interferon-α or interferon-β with PEG, and is for use in subcutaneous administration at an interval of once or twice a week and for use in inhibition of a refractory pleural effusion or ascites accumulation.

Examples of dosage forms of the inhibitory agent for body cavity fluid accumulation for oral administration include tablets, pills, capsules, granules, syrups, emulsions, and suspensions. The dosage forms can be produced in accordance with known techniques, and comprise a carrier or excipient that is generally used in the pharmaceutical field. Examples of carriers and excipients used for tablets include lactose, maltose, saccharose, starch, and magnesium stearate.

Examples of dosage forms of the inhibitory agent for body cavity fluid accumulation for parenteral administration include injection preparations, eye drops, ointments, poultices, suppositories, transnasal absorbents, transpulmonary absorbents, transdermal absorbents, and controlled-release topical agents, and they can be produced in accordance with known techniques. Liquid preparations can be prepared by, for example, dissolving the covalent conjugate of interferon with polyalkylene glycol in a sterile aqueous solution for injection preparations or suspending the conjugate in an extracting solution, and emulsifying to embed the conjugate into a liposome. Solid preparations can be prepared by, for example, adding excipients, such as mannitol, trehalose, sorbitol, lactose, or glucose, to the covalent conjugate of interferon with polyalkylene glycol and preparing lyophilized products. Further, such lyophilized products can be powdered and used. Alternatively, the powders can be mixed with polylactic acid or glycolic acid, and resulting mixture can be solidified and used. Gel agents can be prepared by, for example, dissolving the covalent conjugate of interferon with polyalkylene glycol in a thickener or polysaccharide, such as glycerin, PEG, methylcellulose, carboxy methyl cellulose, hyaluronic acid, or chondroitin sulfate.

Stabilizers, such as human serum albumin, human immunoglobulin, α2 macroglobulin, or an amino acid, can be added to any of the above preparations, and dispersing agents or absorption promoters, such as alcohol, sugar alcohol, ionic surfactant, or nonionic surfactant, can be added, provided that interferon activity is not impaired. Also, trace metals or organic acid salts can be added as appropriate.

### Examples

The present invention is described in greater detail with reference to the following examples, although the technical scope of the present invention is not limited to these examples.

### [Example 1] Preparation of covalent conjugate of interferon-β with PEG

In accordance with the method described in Example 6 of JP Patent No. 4,850,514, the "covalent conjugate of interferon-β with PEG" in which one PEG molecule with a molecular weight of 42,000 is covalently bound to the amino group of lysine at position 134 of the amino acid sequence of recombinant human interferon-β(SEQ ID NO: 1) was prepared. Specifically, ethylene glycol (final concentration: 20%) was added to recombinant human interferon-β (final concentration: 200 µg/ml)that is dissolved in 100 mM acetate buffer (pH 5.0) containing 0.5 M sodium chloride, and the pH was adjusted to 7.6 with 1 M disodium hydrogen phosphate solution. Hydroxysuccinimide ester activated PEG (molecular weight: 42,000; Product No. 61G99122B01; NOF Corporation) was added thereto and mixed, and subjected to a binding reaction at 4°C overnight. To this binding reaction solution, 10 mM acetate buffer (pH 4.5) was added in an amount 5 times greater than the volume thereof, and the resultant was applied to a cation-exchange column (Toyo Pearl CM 650 (S); Tosoh Corporation) equilibrated with 10 mM acetate buffer (pH 4.5). Proteins were eluted and fractionated with developing solvents described below.

### <Developing Solvents>

Solvent A: 10 mM acetate buffer (pH 4.5)
Solvent B: 10 mM acetate buffer (pH 4.5) containing 1 M sodium chloride

While a mixture of Solvent A and Solvent B was allowed to pass through the cation exchange column in an amount 40 times of the amount of the column resin, a ratio of Solvent B for liquid delivery was continuously increased from 0% to 65% to perform elution. The eluted fraction was applied to the SP-5PW column (Tosoh Corporation) and the "covalent conjugate of human interferon-β with PEG" in which one PEG molecule with a molecular weight of 42,000 is covalently bound to the amino group of lysine at position 134 of the amino acid sequence of recombinant human interferon-β(SEQ ID NO: 1) was isolated (hereafter, referred to as "PEG-IFN-β").

In the same manner as with the method for preparing PEG-IFN-β, a "covalent conjugate of mouse interferon-β with PEG" in which one PEG molecule with a molecular weight of 42,000 is covalently bound to recombinant mouse interferon-β was prepared (hereafter, referred to as "PEG-mIFN-β").

Recombinant human interferon-β was prepared in accordance with the method described in Nucleic Acid Research, 1980, Vol. 8, pp. 4057-4074, and recombinant mouse interferon-β was prepared in accordance with the method described in Journal of Interferon Research, 1986, Vol. 6, pp. 519-526.

### [Example 2] Effect of PEG-IFN-β to inhibit ascites accumulation by topical administration

Gastric cancer peritoneal metastasis mouse models were produced, and the effect of PEG-IFN-β to inhibit ascites accumulation by topical administration were evaluated in accordance with the method of Nakanishi et al. (Cancer Science, 2003, Vol. 94, pp. 112-118).

GCIY-EGFP cells prepared by introducing the pEGFP-C1 plasmid (Clontech) into the GCIY human gastric cancer cells (No. RCB0555) obtained from Cell Bank, RIKEN BioResource Center, were cultured and maintained using DMEM medium containing 10% fetal bovine serum in an incubator at 37°C and 5% CO₂. The GCIY-EGFP cells were collected with trypsin/EDTA and washed with a Hank's balanced salt solution (HBSS) to prepare a GCIY-EGFP cell suspension. The GCIY-EGFP cell suspension (2.5 x 10⁶ cells/mouse) was transplanted intraperitoneally to male KSN nude mice (obtained from the Shizuoka Laboratory Animal Center), and whether the GCIY-EGFP cells were engrafted was confirmed on the following day in accordance with the method of Ohashi et al. (International Journal of Oncology, (2005) Vol. 27, pp. 637-644). Mice in which engraftment of the GCIY-EGFP cells was confirmed were subjected to the experiment as gastric cancer peritoneal metastasis mouse models.

Naturally occurring human interferon-β (Feron^{®}; Toray Industries, Inc.) or PEG-IFN-β prepared in Example 1 was administered intraperitoneally (i.e., topically) to gastric cancer peritoneal metastasis mouse models (n = 8) in amounts of 5,000,000 U/mouse/dose. The administration was initiated on the day after transplantation and performed once a week for 4 weeks. Ascites was collected from the gastric cancer peritoneal metastasis mouse models under anesthesia a week after the final administration, and the liquid volume was measured.

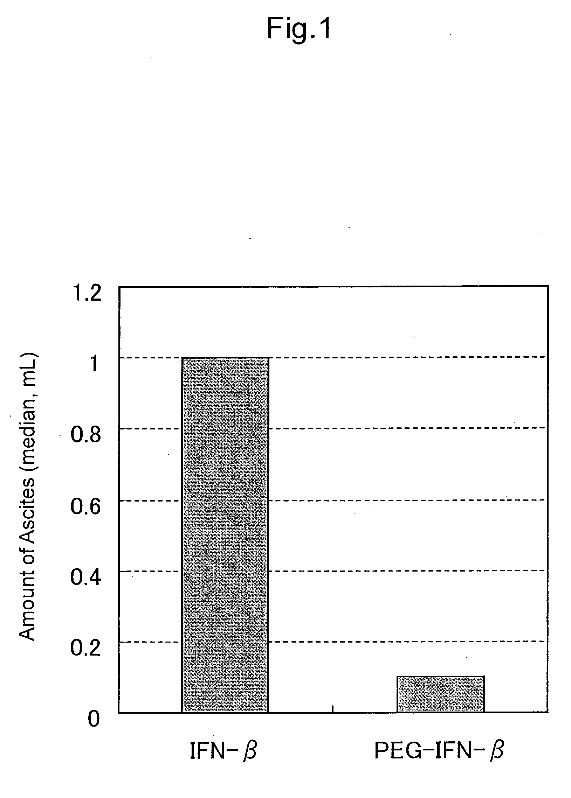

The results are shown in Fig. 1. The vertical axis represents the amount of ascites (median; mL). On the horizontal axis, "IFN-β" represents the result of the group of topical administration of naturally occurring human interferon-β and "PEG-IFN-β" represents the result of the group of topical administration of PEG-IFN-β.

As a result, accumulation of ascites caused by peritoneal metastasis of gastric cancer cells was not inhibited in the group of topical administration of naturally occurring human interferon-β, but accumulation of ascites was inhibited to a normal level in the group of topical administration of PEG-IFN-β. This demonstrated that unmodified interferon-β does not exhibit an effect of inhibiting accumulation of ascites by once-a-week intermittent administration even when it is topically administered at a high dose of 5,000,000 U/mouse/dose into an abdominal cavity to which gastric cancer cells had been metastasized, while the covalent conjugate of interferon-β with PEG exhibits a remarkable effect of inhibiting accumulation of ascites with the same intermittent administration schedule.

The results indicate that the covalent conjugate of interferon-β with polyalkylene glycol has an effect of inhibiting accumulation of ascites and is capable of remarkably inhibiting accumulation of body cavity fluid by topical administration.

### [Example 3] Effect of PEG-IFN-β to inhibit ascites accumulation by systemic administration

Gastric cancer peritoneal metastasis mouse models were produced by transplanting human gastric cancer cells, GCIY-EGFP cells, intraperitoneally into male KSN nude mice in the same manner as in Example 2.

Naturally occurring human interferon-β (Feron^{®}; Toray Industries, Inc.), or PEG-IFN-β or PEG-mIFN-β prepared in Example 1 was administered subcutaneously into the dorsal areas (i.e., via systemic administration) of the gastric cancer peritoneal metastasis mouse models (n = 6) in an amount of 5,000 U/mouse/dose. The administration was initiated on the day after transplantation and performed twice a week for 4 weeks. Phosphate buffer (PBS) was administered to the control group in the same manner. Ascites was collected from the gastric cancer peritoneal metastasis mouse models under anesthesia 2 weeks after the final administration and the liquid volume was measured.

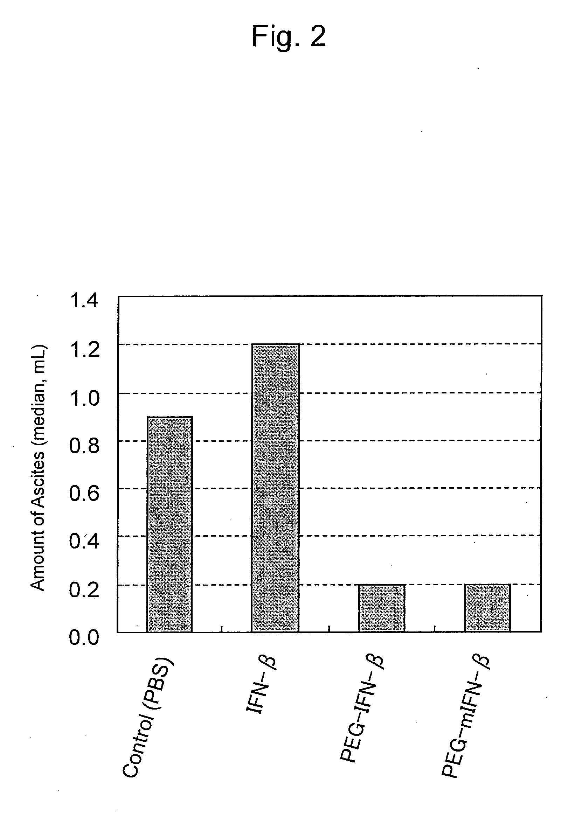

The results are shown in Fig. 2. The vertical axis represents the amount of ascites (median; mL). On the horizontal axis, "Control (PBS)" represents the result of the group of systemic administration of phosphate buffer (PBS) (control group), "IFN-β" represents the result of the group of systemic administration of naturally occurring human interferon-β, "PEG-IFN-β" represents the result of the group of systemic administration of PEG-IFN-β, and "PEG-mIFN-β" represents the result of the group of systemic administration of PEG-mIFN-β.

As a result, the amount of ascites accumulated was found to be greater in the group of systemic administration of naturally occurring human interferon-β than in the group of systemic administration of phosphate buffer (PBS), and thus ascites accumulation caused by gastric cancer peritoneal metastasis was not inhibited in the former group, while accumulation of ascites was remarkably inhibited in both of the group of systemic administration of PEG-IFN-β and the group of systemic administration of PEG-mIFN-β. This demonstrated that unmodified interferon-β does not exhibit any effect of inhibiting accumulation of ascites via systemic administration, while the covalent conjugate of interferon-β with PEG exhibits a remarkable effect of inhibiting accumulation of ascites via systemic administration.

The results indicate that the covalent conjugate of interferon-β with polyalkylene glycol is capable of remarkably inhibiting accumulation of body cavity fluid even when it was administered systemically at a clinical dose on an intermittent schedule.

### [Example 4] Effect of covalent conjugate of interferon-α with PEG to inhibit ascites accumulation by systemic administration

Gastric cancer peritoneal metastasis mouse models were produced by transplanting human gastric cancer cells, GCIY-EGFP cells, intraperitoneally into male KSN nude mice in the same manner as in Example 2.

TS-1^{®} Capsule (hereafter abbreviated as "TS-1") (Taiho Pharmaceutical Co., LTD.) was administered orally to the gastric cancer peritoneal metastasis mouse models at a dose of 0.5 mg of tegafur/mouse/day. The administration was initiated on the day after transplantation and performed daily for 4 weeks.

At the same time, Peginterferon-alfa-2a (covalent conjugate of interferon-α with PEG in which one branched PEG molecule with a molecular weight of about 40,000 is covalently bound to one lysine residue site of recombinant interferon-α-2a via an amide bond) (Pegasys^{®} for subcutaneous injection, Chugai Pharmaceutical Co. Ltd.) was administered subcutaneously into the dorsal areas (i.e., via systemic administration) of the gastric cancer peritoneal metastasis mouse models (n = 6) in an amount of 5,000 U/mouse/dose. The administration was initiated on the day after transplantation and performed twice a week for 4 weeks. As a control, phosphate buffered saline (PBS) was administered subcutaneously (i.e., systemically) to the gastric cancer peritoneal metastasis mouse models twice a week for 4 weeks from the day after transplantation.

Ascites was collected from the gastric cancer peritoneal metastasis mouse models under anesthesia 2 weeks after the final administration, and the liquid volume was measured.

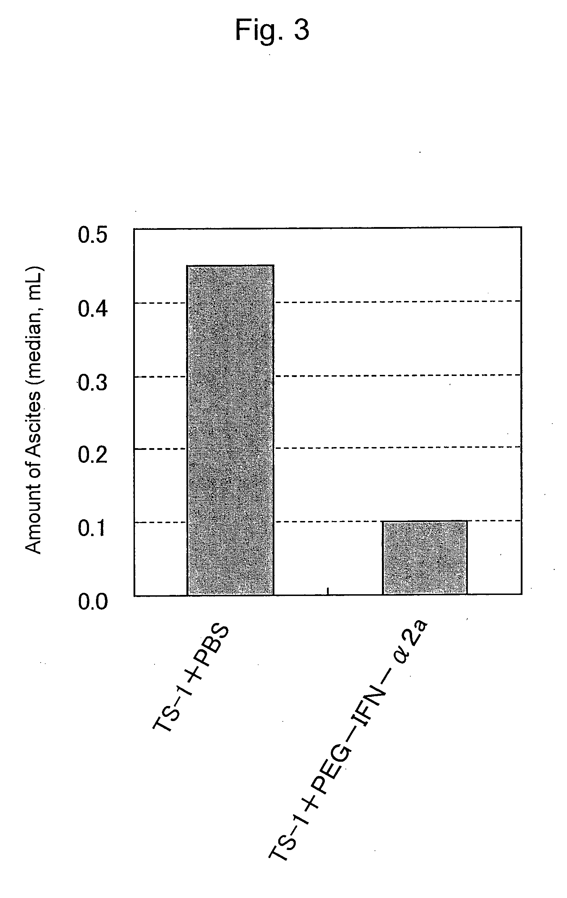

The results are shown in Fig. 3. The vertical axis represents the amount of ascites (median; mL). On the horizontal axis, "TS-1+PBS" represents the result of the group of administration of TS-1 in combination with phosphate buffer (PBS) (i.e., TS-1+PBS group), and "TS-1+PEG-IFN-α2a" represents the result of the group of administration of TS-1 in combination with Peginterferon-alfa-2a (i.e., TS-1+PEG-IFN-α2a group).

As a result, accumulation of ascites caused by peritoneal metastasis of gastric cancer cells was inhibited in the TS-1+PBS group and accumulation of ascites was inhibited to a more remarkable extent in the TS-1+PEG-IFN-α2a group. This demonstrated that the antitumor agent TS-1 exerted inhibitory effects on accumulation of ascites by acting on gastric cancer cells that had metastasized into the abdominal cavity, and simultaneous administration of TS-1 in combination with the covalent conjugate of interferon-α with PEG has a remarkable effect of inhibiting accumulation of ascites.

The results indicate that the covalent conjugate of interferon-α with polyalkylene glycol is capable of remarkably inhibiting body cavity fluid accumulation even when it is administered systemically on an intermittent schedule, and simultaneous administration of the conjugate in combination with an antitumor agent has an effect of inhibiting accumulation of ascites to a more remarkable extent.

### [Example 5] Effects of PEG-IFN-β and PEG-mIFN-β to inhibit reaccumulation of ascites achieved by topical administration

Gastric cancer peritoneal metastasis mouse models were produced using human gastric cancer cells, GCIY cells (RIKEN). GCIY cells were cultured and maintained using MEM medium containing 15% fetal bovine serum in an incubator at 37°C and 5% CO₂.

A GCIY cell suspension (1 x 10⁷ cells/mouse) prepared in the same manner as in Example 2 was transplanted intraperitoneally into male KSN nude mice to produce gastric cancer peritoneal metastasis mouse models. Ascites was collected from the individual mice 5 weeks after the transplantation of the GCIY cell suspension ("initial ascitic paracentesis"). The ascitic paracentesis was performed by puncturing into the abdominal cavity with a winged intravenous needle (18-gauge) under anesthesia and collecting ascites through the needle.

PEG-IFN-β and PEG-mIFN-β prepared in Example 1 were mixed together, the resulting mixture (hereafter, referred to as "PEG-IFN-βs") was administered intraperitoneally (topically) at doses such that PEG-IFN-β and PEG-mIFN-β were each administered in an amount of 10,000 U/mouse/dose, and the administration was initiated on the same day as the initial ascitic paracentesis. The administration was performed at the interval of every other day, which was equivalent to once-a-week administration for humans, and the administration was performed three times in total (n = 4). In the control group, instead of PEG-IFN-βs, the same volume of 20 mM acetate buffer (pH 4.5) containing 150 mM sodium chloride was administered in the same manner (n = 5).

Ascites that had reaccumulated after the initial ascitic paracentesis was collected via ascitic paracentesis from individual mice of the PEG-IFN-βs group and the control group under anesthesia on the day following the day of final administration, and the weight of liquid was measured (second ascitic paracentesis). Individual mice whose ascites had been collected were subjected to laparotomy under anesthesia, the tumor masses that had metastasized to the abdominal cavity were removed with forceps and scissors, and the tumor weights were measured.

Statistical analysis was carried out between two groups; i.e., the control group and the PEG-IFN-βs group, and the Student's t-test or Welch test was selected and carried out on the basis of the test for equality of variance (F-test). Based on the statistical analysis,, the results were determined to be statistically significant when the P value was less than 0.05.

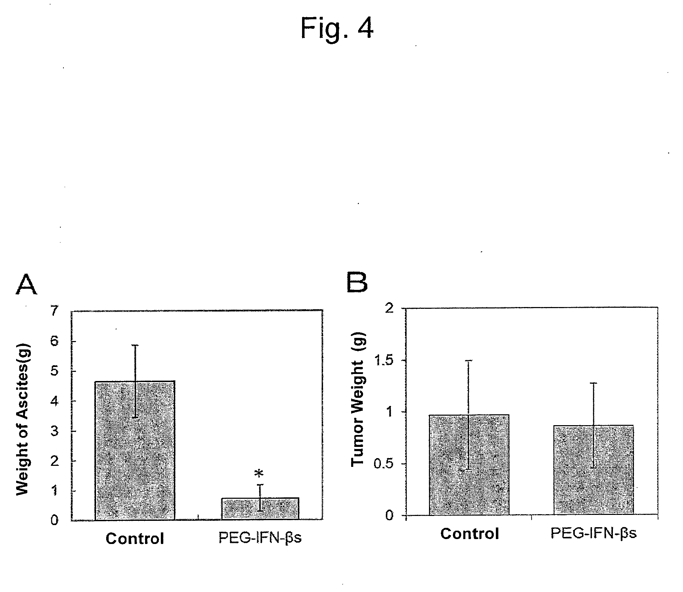

The results are shown in Fig. 4. The vertical axis of Fig. 4A represents the weight of the ascites obtained by the second ascitic paracentesis (n = 4 to 5; mean ± SE), and the vertical axis of Fig. 4B represents the tumor weight (n = 4 to 5; mean ± SE). On the horizontal axis, "Control" represents the group of topical administration of 20 mM acetate buffer (pH 4.5) containing 150 mM sodium chloride (the control group), and "PEG-IFN-βs" represents the group of topical administration of a mixture of PEG-IFN-β and PEG-mIFN-β. The asterisk symbol in the Figure indicates that the data of the group of topical administration of PEG-IFN-β are statistically significant in comparison with the control group (P < 0.05).

As a result, no significant difference was observed in the weights of tumors that had metastasized into the abdominal cavity between the group of topical administration of PEG-IFN-βs and the control group (Fig. 4B), but reaccumulation of ascites caused by peritoneal metastasis was significantly inhibited in the group of topical administration of PEG-IFN-βs, in comparison with the control group (Fig. 4A). This indicates that the covalent conjugate of interferon-β with PEG has also a therapeutic effect via inhibition of recurrence of ascites accumulation even when topically administered at an advanced stage with ascites accumulation due to peritoneal metastasis of gastric cancer cells.

Interferon is known to have an effect of inhibiting cancer cell growth (hereafter, referred to as "antitumor effect"). However, the effects of inhibiting reaccumulation of ascites by administration of PEG-IFN-βs were observed in cases in which their sufficient antitumor effects were not observed. This strongly indicates that the effect of inhibiting reaccumulation of ascites is not a secondary effect due to the antitumor effect of the interferon, but is independent of the antitumor effect.

The results indicate that the covalent conjugate of interferon with polyalkylene glycol exhibits, via topical administration, an effect of inhibiting reaccumulation of body cavity fluid after removal of the body cavity fluid, and such effect is independent of antitumor effects.

### [Example 6] Effect of PEG-IFN-β and PEG-mIFN-β to inhibit reaccumulation of ascites and improve a survival rate by systemic administration

The therapeutic effect of a covalent conjugate of interferon-β with PEG by systemic administration on accumulation of malignant ascites and survival rate was examined.

In accordance with the method of Huynh et al. (Molecular cancer therapeutics, (2007) Vol. 6, pp. 2959-2966), ovarian cancer peritoneal metastasis mouse models were produced, and the effect of PEG-IFN-β to inhibit reaccumulation of ascites by systemic administration was evaluated therewith.

Human ovarian cancer cells OV-90 (CRL-11732; American Type Culture Collection) were cultured and maintained using an MEM medium containing 15% fetal bovine serum in an incubator at 37°C and 5% CO₂. The OV-90 cells were collected with trypsin/EDTA and washed with phosphate buffer (PBS) to prepare an OV-90 cell suspension. The resulting OV-90 cell suspension (5 x 10⁶ cells/mouse) was transplanted intraperitoneally into female SCID mice (C.B.-17/lcr-scid/scid-jcl; CLEA Japan Inc.).

Ascites was collected from the individual mice 46 days after the transplantation of the cell suspension (initial ascitic paracentesis). The ascitic paracentesis was performed by puncturing into the abdominal cavity with a winged intravenous needle (18-gauge) in an awake state and collecting ascites through the needle. The weight of ascites (g) was measured, and individual mice in which ascites accumulation of 0.1 g or more was observed were subjected to the following experiment.

PEG-IFN-β and PEG-mIFN-β prepared in Example 1 were mixed together, the resulting mixture (hereafter, referred to as "PEG-IFN-βs") was administered subcutaneously (systemically) at doses such that PEG-IFN-β and PEG-mIFN-β were each administered in an amount of 50,000 U/mouse/dose, and the administration was initiated on the same day as the initial ascitic paracentesis. The administration was performed at the interval of every other day, which was equivalent to once-a-week administration for humans (n = 6). In the control group, instead of PEG-IFN-βs, the same volume of 20 mM acetate buffer (pH 4.5) containing 150 mM sodium chloride was administered in the same manner (n = 7).

On the day after the completion of the third administration (i.e., 5 days after the initiation of administration), ascites that had reaccumulated after the initial ascitic paracentesis was collected via ascitic paracentesis from individual mice of the PEG-IFN-βs group and the control group in the awake state, and the weight of liquid was measured (the second ascitic paracentesis).

After the second ascitic paracentesis, subsequent administrations were performed subcutaneously (i.e., systemically) three times every other day, and the administration was six times in total. The survival rate of each group from the day on which administration was initiated to 12 days thereafter was examined.

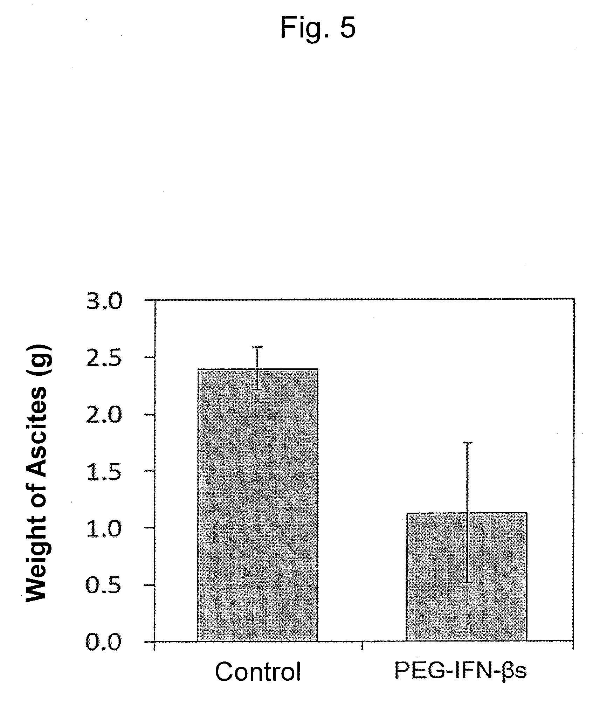

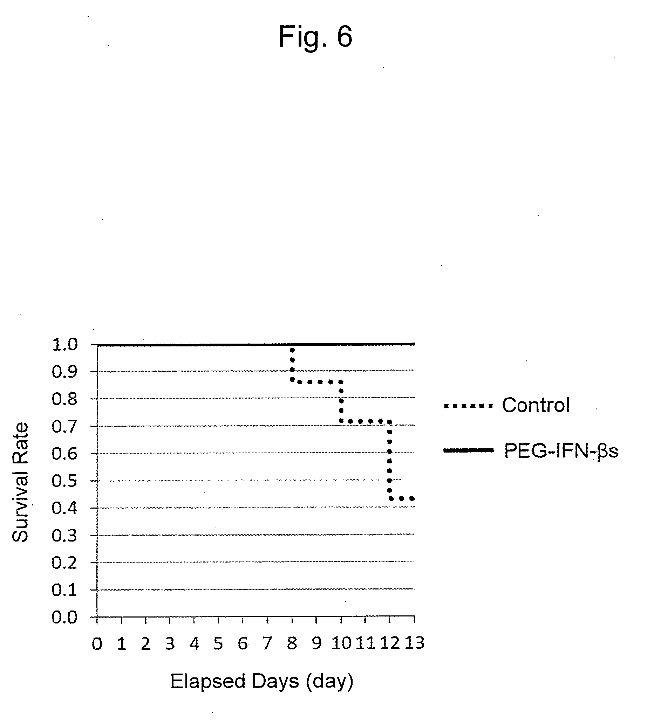

The results are shown in Fig. 5 and Fig. 6. The vertical axis of Fig. 5 represents the weight of the ascites obtained by the second ascitic paracentesis (n = 6 to 7; mean ± SE). The vertical axis of Fig. 6 represents the survival rate, and the horizontal axis represents days after the day on which administration was initiated (day 0). "Control" on the horizontal axis in Fig. 5 and in Fig. 6 represents the group of subcutaneous administration of 20 mM acetate buffer (pH 4.5) containing 150 mM sodium chloride (the control group), and "PEG-IFN-βs" represents the group of subcutaneous administration of a mixture of PEG-IFN-β and PEG-mIFN-β.

As a result, reaccumulation of ascites resulting from peritoneal metastasis was inhibited in the group of systemic administration of PEG-IFN-βs. This indicates that the covalent conjugate of interferon-β with PEG inhibited recurrence of ascites accumulation when systemically administered at an advanced stage with ascites accumulation due to peritoneal metastasis of ovarian cancer cells.

All mice in the group of systemic administration of PEG-IFN-βs had survived up to 12 days after the initiation of administration. In the control group, however, a death of mouse was first observed 8 days after the initiation of administration, and the survival rate was less than 50% 12 days after the initiation of administration. This indicates that the covalent conjugate of interferon-β with PEG inhibits aggravation of generalized condition and improves survival rate when systemically administered at an advanced stage with ascites accumulation due to peritoneal metastasis of ovarian cancer cells.

The results indicate that the covalent conjugate of interferon with polyalkylene glycol exhibits, even via systemic administration, an effect of inhibiting reaccumulation of body cavity fluid after removal of the body cavity fluid. Also, they show that the covalent conjugate inhibits aggravation of generalized condition and improves survival rate when systemically administered at an advanced cancer stage with accumulation of body cavity fluid.

The Examples above demonstrate that a covalent conjugate of interferon with polyalkylene glycol (preferably, a covalent conjugate of interferon-α or interferon-β with PEG) enables inhibition of a refractory ascites accumulation, which could not be realized with interferon-α or interferon-β. More specifically, it is revealed that a covalent conjugate of interferon with polyalkylene glycol (preferably, a covalent conjugate of interferon-α or interferon-β with PEG) has an effect of inhibiting accumulation of malignant ascites via systemic administration such as subcutaneous administration or topical administration on an intermittent schedule in which the administration is carried out at an interval of once every two or more days. Further, it is shown that the inhibitory effect is not a secondary effect due to antitumor effects, and such effects are exerted regardless of cancer type. It is also shown that, in comparison with conventional treatment carried out via daily administration of interferon, treatment via administration of the covalent conjugate of interferon with polyalkylene glycol on an intermittent schedule exhibits an enhanced therapeutic effect on malignant ascites.

### Industrial Applicability

The present invention can be used as an inhibitory agent for body cavity fluid accumulation in the pharmaceutical field.

### Sequence Listing Free Text

SEQ ID NO: 1: the amino acid sequence of human interferon-β

## Claims

1. An inhibitory agent for body cavity fluid accumulation comprising, as an active ingredient, a covalent conjugate of interferon with polyalkylene glycol.

2. The inhibitory agent for body cavity fluid accumulation according to claim 1, wherein the polyalkylene glycol is polyethylene glycol.

3. The inhibitory agent for body cavity fluid accumulation according to claim 1 or 2, wherein the interferon is type I interferon or interferon-λ.

4. The inhibitory agent for body cavity fluid accumulation according to claim 3, wherein the type I interferon is interferon-α or interferon-β.

5. The inhibitory agent for body cavity fluid accumulation according to any one of claims 1 to 4, which inhibits accumulation of a pleural effusion or ascites.

6. The inhibitory agent for body cavity fluid accumulation according to claim 5, wherein the pleural effusion or ascites is a refractory pleural effusion or ascites accumulated due to cancer, cirrhosis, or renal failure.

7. The inhibitory agent for body cavity fluid accumulation according to any one of claims 1 to 6, which is used for systemic administration.

8. The inhibitory agent for body cavity fluid accumulation according claim 7, wherein the systemic administration is selected from the group consisting of subcutaneous administration, intracutaneous administration, intramuscular administration, intravenous administration, and intraarterial administration.

9. The inhibitory agent for body cavity fluid accumulation according to any one of claims 1 to 8, which is used in combination with an antitumor agent.

10. The inhibitory agent for body cavity fluid accumulation according to claim 9, wherein the antitumor agent is at least one antitumor agent selected from the group consisting of 5-FU, tegafur-uracil, tegafur-gimeracil-oteracil, and capecitabine.


SEQUENCE LISTING

<110>  Toray Industries, Inc.

<120>  Inhibitor of the accumulation of body cavity fluid

<130>  13021WO01

<160>  1

<170>  PatentIn version 3.1

<210>  1
<211>  166
<212>  PRT
<213>  Homo sapiens

<400>  1

Met Ser Tyr Asn Leu Leu Gly Phe Leu Gln Arg Ser Ser Asn Phe Gln
1               5                   10                  15

Cys Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Leu Gln
        35                  40                  45

Gln Phe Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165

SEQUENCE LISTING

<110>  Toray Industries, Inc.

<120>  Inhibitor of the accumulation of body cavity fluid

<130>  13021WO01

<160>  1

<170>  PatentIn version 3.1

<210>  1
<211>  166
<212>  PRT
<213>  Homo sapiens

<400>  1

Met Ser Tyr Asn Leu Leu Gly Phe Leu Gln Arg Ser Ser Asn Phe Gln
1               5                   10                  15

Cys Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Leu Gln
        35                  40                  45

Gln Phe Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165